# EUROPEAN PATENT SPECIFICATION

(11) **EP 4 456 868 B1**
(45) Date of publication and mention of the grant of the patent: **02.04.2025**
(21) Application number: 22840632.8
(22) Date of filing: 19.12.2022
(51) Int. Cl.: A61K 8/44, A61K 8/9789, A61Q 19/08

(54) **PERSONAL CARE COMPOSITION**
KÖRPERPFLEGEZUSAMMENSETZUNG
COMPOSITION DE SOINS PERSONNELS

(30) Priority: 31.12.2021 WO PCT/CN2021/143516; 27.01.2022 EP 22153548
(43) Date of publication of application: 06.11.2024
(73) Proprietor: Unilever IP Holdings B.V., 3013 AL Rotterdam (NL); Unilever Global IP Limited, Wirral, Merseyside CH62 4ZD (GB)
(72) Inventor: GU, Xuelan, 6708 WH Wageningen (NL); MI, Tingyan, 6708 WH Wageningen (NL)
(74) Representative: James, Helen Sarah
(86) International application number: PCT/EP2022/086751
(87) International publication number: WO 2023/126238

(56) References cited:
- CN-A- 109 316 393
- KR-B1- 101 220 903
- US-A- 6 153 649

## Description

### Field of the Invention

The present invention relates to a personal care composition comprising a carboxymethyl cysteine compound and *Sandalwood* extract for anti-aging.

### Background of the Invention

Aging is an inevitable progression of life. Consumers seek to alleviate or delay the signs of chronological aged or photoaged skin, such as fine lines and wrinkles, dry and sagging skin. Various strategies have been developed to achieve the anti-aging effects for consumers.

Hyaluronic acid is found in skin not only in the dermis but also in the epidermal intercellular spaces. It can bind and retain huge amount of water and may help moisturize and firm the skin. The content of hyaluronic acid decreases with age, which is one factor believed to account for the change in aged skin, such as loss of skin moisture, as well as the development of wrinkles and fine lines. Therefore, it is desired to boost the synthesis stimulate and the function of hyaluronic acid.

HAS3 (hyaluronan synthase 3) plays an important role in regulating synthesis of hyaluronic acid in the epidermis. The interaction of hyaluronic acid with CD44 cell surface glycoprotein is a driver of collagen synthesis and other hyaluronic acid mediated skin effects, like proliferation of keratinocytes. The synthesis of hyaluronic acid and collagen in the skin as well as other aging processes in the skin may be improved by elevating the expression of HAS3 and CD44. The present inventors have recognized such needs and have developed a personal care composition comprising carboxymethyl cysteine compound and *Sandalwood* extract. It was surprisingly found that by combining carboxymethyl cysteine compound with *Sandalwood* extract, the expression of both HAS3 and CD44 are significantly elevated.

KR101220903 discloses a cosmetic composition for skin wrinkles comprising sandalwood extract as an active ingredient for skin wrinkles.

### Summary of the Invention

In a first aspect, the present invention is directed to a personal care composition comprising carboxymethyl cysteine compound and *Sandalwood* extract.

In a second aspect, the present invention is directed to a method of improving skin moisturization; treating, reducing, and/or preventing of fine lines or wrinkles of skin; improving collagen production in skin; providing anti-aging benefit to skin; and/or firming skin, comprising a step of topically applying to the skin the composition of the present invention.

In a third aspect, the present invention is directed to use of the composition of the present invention for improving skin moisturization; treating, reducing, and/or preventing of fine lines or wrinkles of skin; improving collagen production in skin; providing anti-aging benefit to skin; and/or firming skin.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The carboxymethyl cysteine compound refers to compound selected from carboxymethyl cysteine, salt of carboxymethyl cysteine, ester of carboxymethyl cysteine, amide of carboxymethyl cysteine or a mixture thereof. Preferably, the carboxymethyl cysteine compound comprises carboxymethyl cysteine, ester of carboxymethyl cysteine, and/or salt of carboxymethyl cysteine. More preferably, the carboxymethyl cysteine compound comprises carboxymethyl cysteine, and/or salt of carboxymethyl cysteine. Even more preferably, carboxymethyl cysteine compound comprises salt of carboxymethyl cysteine. Still even more preferably the carboxymethyl cysteine compound comprises lysine carboxymethyl cysteinate and most preferably, the carboxymethyl cysteine compound is lysine carboxymethyl cysteinate.

Preferably, the carboxymethyl cysteine compound is present in amount of at least 0.00001%, more preferably at least 0.0001%, even more preferably at least 0.001%, still even more preferably at least 0.01%, and most preferably at least 0.1% by weight of the composition. Preferably, the carboxymethyl cysteine compound is present in amount of no greater than 10%, more preferably no greater than 5%, even more preferably no greater than 3%, still even more preferably no greater than 1%, and most preferably no greater than 0.5% by weight of the composition.

Preferably, the lysine carboxymethyl cysteinate is present in amount of no greater than 10%, more preferably no greater than 5%, even more preferably no greater than 3%, still even more preferably no greater than 1%, and most preferably no greater than 0.5% by weight of the composition. Preferably, the lysine carboxymethyl cysteinate is present in amount of at least 0.00001%, more preferably at least 0.0001%, even more preferably at least 0.001%, still even more preferably at least 0.01%, and most preferably at least 0.1% by weight of the composition.

The composition of the present invention comprises *Sandalwood* (*Santalum album*) extract. Preferably the *Sandalwood* extract according to the invention is obtained from wood of the *Sandalwood.* Preferably, the *Sandalwood* extract comprises extract having INCI (International Nomenclature Cosmetic Ingredient) name of *Sandalwood* oil and/or *Sandalwood* wood extract. More preferably, the *Sandalwood* extract comprises extract having INCI name of *Sandalwood* wood extract. Most preferably, the *Sandalwood* extract is extract having INCI name of *Sandalwood* wood extract.

Preferably, the *Sandalwood* extract is obtainable by extracting *Sandalwood* using a supercritical fluid. Preferably the *Sandalwood* extract is obtainable by extracting a *Sandalwood* using a supercritical carbon dioxide. Preferably, the *Sandalwood* extract is obtainable by extracting a *Sandalwood* wood using a supercritical carbon dioxide. Preferably the extraction is carried out using a fluid in the supercritical state, preferably carbon dioxide and/or xenon, in the presence of a co-solvent chosen from primary or secondary alcohols. Preferably the extraction temperature is 35 to 85 °C, more preferably 45 to 75 °C, and most preferably 55 to 65 °C. The pressure within the extractor is typically 90 to 1000 bar, preferably 150 to 700 bar, and more preferably 250 to 400 bar.

Preferably, the *Sandalwood* extract is present in amounts of 0.00001 to 5% by weight of the composition, more preferably in amounts of 0.0001 to 1%, even more preferably 0.0005 to 0.2%, still even more preferably 0.001 to 0.1% by weight of the total composition. Preferably the *Sandalwood* wood extract is present in amounts of 0.00001 to 5% by weight of the composition, more preferably in amounts of 0.0001 to 1%, even more preferably 0.0005 to 0.2%, still even more preferably 0.001 to 0.1% by weight of the total composition.

For sake of clarity, the amount of extract in the present invention refers to the amount of extracted ingredients from the plant, excluding the weight of the extract solvent.

Preferably, the weight ratio of the carboxymethyl cysteine compound to the *Sandalwood* extract is 2:1 to 5000:1, more preferably 3:1 to 1500:1, even more preferably 10:1 to 600:1 and still even more preferably 40:1 to 150:1. Preferably, the weight ratio of the lysine carboxymethyl cysteinate to the *Sandalwood* extract is 2:1 to 5000:1, more preferably 3:1 to 1500:1, even more preferably 10:1 to 600:1 and still even more preferably 40:1 to 150:1. Preferably, the weight ratio of the lysine carboxymethyl cysteinate to the *Sandalwood* wood extract is 2:1 to 5000:1, more preferably 3:1 to 1500:1, even more preferably 10:1 to 600:1 and still even more preferably 40:1 to 150:1.

Preferably, the composition comprises Vitamin B3 compounds (including derivatives of vitamin B3). The vitamin B3 compounds comprises niacin, nicotinic acid, niacinamide or a mixture thereof. The most preferred vitamin B3 compound is niacinamide. Amount of Vitamin B3 compounds may be 0.1 to 10%, preferably 0.5 to 5% by weight of the composition.

Preferably, the composition comprises a glutamate source selected from the group consisting of glutamine, glutamine ester, glutamic acid, pyroglutamic acid, salts, and mixtures thereof. More preferably, the composition comprises pyroglutamic acid and/or salt of pyroglutamic acid. Even more preferably, the composition comprises sodium salt of pyroglutamic acid. Preferably, the glutamate source is present in amount of 0.0001 to 10% by weight of the composition, more preferably 0.001 to 6%, even more preferably 0.01 to 3% by weight of the composition.

The composition may optionally comprise whitening pigment. Whitening pigments are typically particles of high refractive index materials. For example, the whitening pigment may have a refractive index of greater than 1.3, more preferably greater than 1.8 and most preferably from 2.0 to 2.7. Examples of such whitening pigment are those comprising bismuth oxy-chloride, boron nitride, barium sulfate, mica, silica, titanium dioxide, zirconium oxide, aluminium oxide, zinc oxide or combinations thereof. More preferred whitening pigment are particles comprising titanium dioxide, zinc oxide, zirconium oxide, mica, iron oxide or a combination thereof. Even more preferred whitening pigment are particles comprising zinc oxide, zirconium oxide, titanium dioxide or a combination thereof as these materials have especially high refractive index. Still even more preferably the whitening pigment is selected from titanium dioxide, zinc oxide or a mixture thereof and most preferred whitening pigment is titanium dioxide. The average diameter of whitening pigment is typical from 15 nm to 1 micron, more preferably from 35 nm to 800 nm, even more preferably from 50 nm to 500 nm and still even more preferably from 100 to 300 nm. Amount of whitening pigment may be 0.1 to 15%, preferably 0.5 to 5% by weight of the composition.

Preferably, the composition comprises polyhydric alcohol. Polyhydric alcohols may be selected from group of glycerin, propylyene glycol, dipropylene glycol, polypropylene glycol, polyethylene glycol, sorbitol, hydroxypropyl sorbitol, hexylene glycol, 1,3-butylene glycol, isoprene glycol, ethoxylated glycerol, propoxylated glycerol or a mixture thereof. Most preferred polyhydric alcohol is glycerol known also as glycerin. The amount of polyhydric alcohol may range anywhere from 0.1 to 20%, preferably 0.5 to 15% and more preferably 2 and 10% by weight of the composition.

Preferably, the composition comprises emollient materials. Suitable emollient materials include silicones, hydrocarbons, triglycerides or a mixture thereof. These silicones may be organic, silicone-containing or fluorine-containing, volatile or non-volatile, polar or non-polar. Hydrocarbons may include mineral oil, petrolatum and polyalpha-olefins. Examples of preferred volatile hydrocarbons include polydecanes such as isododecane and isodecane (e.g. Permethyl-99A which is available from Presperse Inc.) and the C7-C8 through C12-C15 isoparaffins (such as the Isopar Series available from Exxon Chemicals). Illustrative triglycerides but not limiting are sunflower seed oil, cotton oil, canola oil, soybean oil, castor oil, borage oil, olive oil, shea butter, jojoba oil and mixtures thereof. Mono- and di- glycerides may also be useful. Particularly preferable are glyceryl monostearate and glyceryl distearate.

Preferably, the composition comprises moisturizing agents. Particularly preferred moisturizing agents includes, petrolatum, aquaporin manipulating actives, oat kernel flour, substituted urea like hydroxyethyl urea, hyaluronic acid and/or its precursor N-acetyl glucosamine, hyaluronic acid and/or its precursor N-acetyl glucosamine, or a mixture thereof.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 96% by weight of the composition, more preferably from 25 to 92%, even more preferably from 42 to 88%, most preferably from 55 to 82% by weight of the composition.

Preferably, the composition has a viscosity of at least 10 mPa·s, more preferably in the range 30 to 10000 mPa·s, even more preferably 50 to 5000 mPa·s, and most preferably 100 to 2000 mPa·s, when measured at 20 degrees C at a relatively high shear rate of about 20 s⁻¹. Preferably, the composition is in the form of fluid.

Preferably, the composition is an emulsion, more preferably an oil-in-water emulsion. The skin care composition refers to a composition suitable for topical application to human skin, including leave-on and wash-off products but preferably leave-on compositions. The term "leave-on" as used with reference to compositions herein means a composition that is applied to or rubbed on the skin, and left thereon. The term "wash-off" as used with reference to compositions herein means a skin cleanser that is applied to or rubbed on the skin and rinsed off substantially immediately subsequent to application. Preferably the skin care composition means a fluid liquid, and particularly a moisturizer rather than a make-up product.

Preferably, the personal care composition is a skin care composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

Preferably the use is non-therapeutic. Preferably the method is non-therapeutic. The term non-therapeutic typically means for cosmetic purposes and not curative or therapeutic purposes.

The following examples are provided to facilitate an understanding of the invention. The examples are not intended to limit the scope of the claims.

### Examples

### Materials

| Supplier | Trade name | Active | Abbreviation | Active wt% |
|---|---|---|---|---|
| Sinerga | HAIR APP | Lysine carboxymethyl cysteinate | LCC | >98% |
| Ashland | Santalwood^{™} | *Sandalwood* extract* | - | 0.5% |

| | | | | |
|---|---|---|---|---|
| * The sandalwood extract was obtained from Ashland (China) Investing Company. The sandalwood for preparing the Sandalwood extract was obtained in Australia. | | | | |

### Example 1

This Example demonstrates the improved anti-aging effect by combining lysine carboxymethyl cysteinate and *Sandalwood* extract.

The normal human epidermal keratinocytes (NHEK) (Biocell, Xi'an, China) were incubated in medium together with or without actives for 24 hours. After incubation, the total RNA for each NHEK was extracted using RNAiso Plus (TaKaRa, Cat: 9108; Cat: RR036) according to manufacturer's protocol.

The extracted RNA was quantified using Nanodrop spectrometer (Thermo Fisher Scientific, Waltham, MA, US) and reverse transcribed to generate the template cDNA using the PrimeScript II 1st Strand cDNA synthesis kit according to manufacturer's protocol. Amplification of the gene encoding the hyaluronan synthase 3 (HAS3) and CD44 was performed with RT-PCR reagent (SYBR^{®} Premix Ex Taq^{™}) on the ABI Vii 7 Real-Time PCR Systems (Applied Biosystems, Thermo fisher scientific, Carlsbad, CA, USA). The beta-actin gene was selected as the housekeeping gene and all data on relative expression of the target genes was normalized to beta-actin. The fold changes in expression were calculated relative to the blank control (medium having no active). All tests were conducted at least three times and Table 1 shows the fold changes in expression of HAS3 and CD44.

**Table 1**

| NHEK | Incubated in ^{#} | HAS3 expression | CD44 expression |
|---|---|---|---|
| 1 (Blank control) | Medium | 1.02 | 1.03 |
| 2 | 0.02 wt% of Santalwood^{™} in medium | 1.87 | 2.45 |
| 3 | 0.05 wt% of LCC in medium | 0.84 | 1.06 |
| 4 | 0.02 wt% of Santalwood^{™} and 0.05 wt% of LCC in medium | 3.2 * | 3.14 * |

| | | | |
|---|---|---|---|
| #: The level of the active is based on the medium. * Significantly elevated (p<0.05) than any of single active. | | | |

As demonstrated in Table 1, it was surprisingly found that both HAS3 expression and CD44 expression were significantly elevated by combining lysine carboxymethyl cysteinate and *Sandalwood* extract.

## Claims

1. A personal care composition comprising carboxymethyl cysteine compound and *Sandalwood* extract.

2. The composition according to claim 1 wherein the carboxymethyl cysteine compound comprises carboxymethyl cysteine, ester of carboxymethyl cysteine, and/or salt of carboxymethyl cysteine.

3. The composition according to claim 2 wherein the carboxymethyl cysteine compound comprises salt of carboxymethyl cysteine and preferably the carboxymethyl cysteine compound comprises lysine carboxymethyl cysteinate.

4. The composition according to any one of the preceding claims wherein the carboxymethyl cysteine compound is present in amount of at least 0.00001% and no greater than 10% by weight of the composition, preferably carboxymethyl cysteine compound is present in amount of at least 0.01% and no greater than 3% by weight of the composition.

5. The composition according to any one of the preceding claims wherein the *Sandalwood* extract comprises extract having INCI name of *Sandalwood* oil and/or *Sandalwood* wood extract, preferably the *Sandalwood* extract is *Sandalwood* wood extract.

6. The composition according to any one of the preceding claims wherein the *Sandalwood* extract is obtainable by extracting *Sandalwood* using a supercritical fluid, preferably a supercritical carbon dioxide.

7. The composition according to any one of the preceding claims wherein the *Sandalwood* extract is present in amounts of 0.00001 to 5% by weight of the composition, preferably 0.001 to 0.1% by weight of the total composition.

8. The composition according to any one of the preceding claims wherein the weight ratio of the carboxymethyl cysteine compound to the *Sandalwood* extract is 2:1 to 5000:1, preferably 10:1 to 600:1 and more preferably 40:1 to 150:1.

9. The composition according to any one of the preceding claims 5 to 8 wherein the weight ratio of the lysine carboxymethyl cysteinate to the *Sandalwood* extract is 2:1 to 5000:1 preferably 40:1 to 150:1.

10. The composition according to any one of the preceding claims wherein the composition is an emulsion, preferably an oil-in-water emulsion.

11. The composition according to any one of the preceding claims wherein the composition comprises water in amount of 10 to 96% by weight of the composition, preferably from 42 to 88% by weight of the composition.

12. A method of improving skin moisturization; treating, reducing, and/or preventing of fine lines or wrinkles of skin; improving collagen production in skin; providing anti-aging benefit to skin; and/or firming skin, comprising a step of topically applying to the skin the composition of any one of the preceding claims.

13. Use of the composition of any one of the preceding claims 1 to 11 for improving skin moisturization; treating, reducing, and/or preventing of fine lines or wrinkles of skin; improving collagen production in skin; providing anti-aging benefit to skin; and/or firming skin.

## Patentansprüche

1. Körperpflegezusammensetzung, die eine Carboxymethylcystein-Verbindung und Sandelholz-Extrakt umfasst.

2. Zusammensetzung nach Anspruch 1, wobei die Carboxymethylcystein-Verbindung Carboxymethylcystein, Ester von Carboxymethylcystein und/oder Salz von Carboxymethylcystein umfasst.

3. Zusammensetzung nach Anspruch 2, wobei die Carboxymethylcystein-Verbindung Salz von Carboxymethylcystein umfasst und die Carboxymethylcy-stein-Verbindung bevorzugt Lysin-Carboxymethylcysteinat umfasst.

4. Zusammensetzung nach irgendeinem der vorhergehenden Ansprüche, wobei die Carboxymethylcystein-Verbindung in einer Menge von mindestens 0,00001 Gewichts-% und von nicht mehr als 10 Gewichts-% der Zusammensetzung vorliegt, wobei es bevorzugt ist, dass die Carboxymethylcystein-Verbindung in einer Menge von mindestens 0,01 und von nicht mehr als 3 Gewichts-% der Zusammensetzung vorliegt.

5. Zusammensetzung nach irgendeinem der vorhergehenden Ansprüche, wobei der Sandelholz-Extrakt den Extrakt mit der INCI-Bezeichnung Sandelholzöl und/oder Sandelholz-Holzextrakt umfasst, wobei der Sandelholz-Extrakt bevorzugt Sandelholz-Holzextrakt ist.

6. Zusammensetzung nach irgendeinem der vorhergehenden Ansprüche, wobei der Sandelholz-Extrakt durch Extraktion von Sandelholz unter Verwendung einer überkritischen Flüssigkeit, bevorzugt eines überkritischen Kohlendioxids, erhältlich ist.

7. Zusammensetzung nach irgendeinem der vorhergehenden Ansprüche, wobei der Sandelholz-Extrakt in Mengen von 0,00001 bis 5 Gewichts-% der Zusammensetzung, bevorzugt von 0,001 bis 0,1 Gewichts-% der Gesamtzusammensetzung, vorliegt.

8. Zusammensetzung nach irgendeinem der vorhergehenden Ansprüche, wobei das Gewichtsverhältnis der Carboxymethylcystein-Verbindung zu dem Sandelholz-Extrakt 2:1 bis 5000:1, bevorzugt 10:1 bis 600:1 und bevorzugter 40:1 bis 150:1 beträgt.

9. Zusammensetzung nach irgendeinem der vorhergehenden Ansprüche 5 bis 8, wobei das Gewichtsverhältnis des Lysin-Carboxymethylcysteinats zu dem Sandelholz-Extrakt 2:1 bis 5000:1, bevorzugt 40:1 bis 150:1, beträgt.

10. Zusammensetzung nach irgendeinem der vorhergehenden Ansprüche, wobei die Zusammensetzung eine Emulsion ist, bevorzugt eine Öl-in-Wasser-Emulsion.

11. Zusammensetzung nach irgendeinem der vorhergehenden Ansprüche, wobei die Zusammensetzung Wasser in einer Menge von 10 bis 96 Gewichts-% der Zusammensetzung, bevorzugt von 42 bis 88 Gewichts-% der Zusammensetzung, umfasst.

12. Verfahren zur Verbesserung der Hautbefeuchtung; zur Behandlung, Verringerung und/oder Vorbeugung von feinen Linien und Fältchen der Haut; zur Verbesserung der Kollagenproduktion in der Haut; zur Erzeugung einer Anti-Aging-Wirkung auf der Haut; und/oder zur Straffung der Haut, umfassend einen Schritt des topischen Auftragens der Zusammensetzung nach einem der vorhergehenden Ansprüche auf die Haut.

13. Verwendung der Zusammensetzung nach irgendeinem der vorhergehenden Ansprüche 1 bis 11 zur Verbesserung der Hautbefeuchtung; zur Behandlung, Verringerung und/oder Vorbeugung von feinen Linien oder Fältchen der Haut, zur Verbesserung der Kollagenproduktion in der Haut; zur Erzeugung einer Anti-Aging-Wirkung auf der Haut und/oder zur Straffung der Haut.

## Revendications

1. Composition de soin personnel comprenant un composé de carboxyméthylcystéine et un extrait de santal.

2. Composition selon la revendication 1, dans laquelle le composé de carboxyméthylcystéine comprend la carboxyméthylcystéine, un ester de carboxyméthylcystéine, et/ou un sel de carboxyméthylcystéine.

3. Composition selon la revendication 2, dans laquelle le composé de carboxyméthylcystéine comprend un sel de carboxyméthylcystéine et de préférence le composé de carboxyméthylcystéine comprend du carboxyméthylcystéinate de lysine.

4. Composition selon l'une quelconque des revendications précédentes, dans laquelle le composé de carboxyméthylcystéine est présent en une quantité d'au moins 0,00001 % et non supérieure à 10 % en poids de la composition, de préférence le composé de carboxyméthylcystéine est présent en une quantité d'au moins 0,01 % et non supérieure à 3 % en poids de la composition.

5. Composition selon l'une quelconque des revendications précédentes, dans laquelle l'extrait de santal comprend un extrait ayant le nom INCI d'essence de santal et/ou d'extrait de bois de santal, de préférence l'extrait de santal est un extrait de bois de santal.

6. Composition selon l'une quelconque des revendications précédentes, dans laquelle l'extrait de santal peut être obtenu par extraction de santal au moyen d'un fluide supercritique, de préférence de dioxyde de carbone supercritique.

7. Composition selon l'une quelconque des revendications précédentes, dans laquelle l'extrait de santal est présent en des quantités de 0,00001 à 5 % en poids de la composition, de préférence de 0,001 à 0,1 % en poids de la composition totale.

8. Composition selon l'une quelconque des revendications précédentes, dans laquelle le rapport en poids du composé de carboxyméthylcystéine à l'extrait de santal est de 2/1 à 5000/1, de préférence de 10/1 à 600/1 et mieux encore de 40/1 à 150/1.

9. Composition selon l'une quelconque des revendications 5 à 8, dans lequel le rapport en poids du carboxyméthylcystéinate de lysine à l'extrait de santal est de 2/1 à 5000/1, de préférence de 40/1 à 150/1.

10. Composition selon l'une quelconque des revendications précédentes, dans laquelle la composition est une émulsion, de préférence une émulsion huile dans l'eau.

11. Composition selon l'une quelconque des revendications précédentes, dans laquelle la composition comprend de l'eau en une quantité de 10 à 96 % en poids de la composition, de préférence de 42 à 88 % en poids de la composition.

12. Procédé pour améliorer l'hydratation de la peau ; traiter, réduire et/ou prévenir les ridules ou les rides de la peau ; améliorer la production de collagène dans la peau ; conférer un bénéfice antiâge à la peau ; et/ou raffermir la peau, comprenant une étape d'application topique à la peau de la composition de l'une quelconque des revendications précédentes.

13. Utilisation de la composition de l'une quelconque des revendications 1 à 11 pour améliorer l'hydratation de la peau ; traiter, réduire et/ou prévenir les ridules ou les rides de la peau ; améliorer la production de collagène dans la peau ; conférer un bénéfice antiâge à la peau ; et/ou raffermir la peau.
